Europäisches Patentamt

European Patent Office

Office européen des brevets

(11) Publication number: **0 152 686**
**B1**

# EUROPEAN PATENT SPECIFICATION

(45) Date of publication of patent specification: **15.11.89**

(51) Int. Cl.⁴: **A 61 B 17/36**

(21) Application number: **84308407.0**

(22) Date of filing: **04.12.84**

(54) Laser incisional device.

(30) Priority: **19.01.84 US 571827**

(43) Date of publication of application:
**28.08.85 Bulletin 85/35**

(45) Publication of the grant of the patent:
**15.11.89 Bulletin 89/46**

(84) Designated Contracting States:
**BE CH DE FR GB IT LI NL**

(56) References cited:
**US-A-3 327 712**
**US-A-3 467 098**
**US-A-3 821 510**
**US-A-3 865 113**

(73) Proprietor: **L'Esperance, Francis A.**
**255 Oakwood Road**
**Englewood New Jersey 07631 (US)**

(72) Inventor: **L'Esperance, Francis A.**
**255 Oakwood Road**
**Englewood New Jersey 07631 (US)**

(74) Representative: **Milhench, Howard Leslie et al**
**R.G.C. Jenkins & Co. 26 Caxton Street**
**London SW1H 0RJ (GB)**

Courier Press, Leamington Spa, England.

## Description

The present invention relates to laser surgery and in particular to a tool which is sufficiently portable to enable manual manipulation of laser radiation in the performance of a surgical operation.

Laser surgery to date has involved relatively bulky YAG apparatus wherein relatively great output power density dictates short exposure, and wherein the part of a body to be operated upon must be precisely positioned for the particular desired operation. Radiation wavelengths are in the near-infrared and therefore local burning is a necessary consequence of the exposure.

In copending European Patent application No. 84307972.4 (Serial No. EP-A-0151869), there is disclosed laser apparatus and techniques for ophthalmological surgery wherein selective ablation of body cells is achieved by photodecomposition, without burning, using radiation limited essentially to the ultraviolet end of the spectrum, and reference is made to said application for a more complete discussion.

It is an object of the present invention to provide a readily manipulable surgical tool, as in the manipulable nature of a scalpel, whereby laser radiation may be selectively applied for surgical purposes.

From US-A-3 467 098 there is already known tool for use in laser surgery, said tool being adapted to be connected to laser means having a radiation-beam output by means of an elongate flexible optical-fibre cable coupled at one end to said output and at the other end to said tool, said tool having a body with a cavity bore to one end of which the said other end of said cable is affixed in use for discharge of the laser output into the cavity bore of the tool, and optical element means mounted in said bore for establishing a focussed beam of laser radiation directed towards the other end of the bore. From US-A-3 821 510 there is further known such a tool wherein the optical element means provides a collimated beam of laser radiation and the tool further comprises a lens mount separate from but attachable to the body at the other end of the bore and incorporating lens means for focussing said beam of laser radiation.

In accordance with the present invention there is provided a tool as above-defined, known from US-A-3 821 510, which is characterized in accordance with the invention in that said lens means comprises a cylindrical lens mounted with its axis transverse to the axis of said bore for focussing the beam of laser radiation into an elongate region transverse to said axis and longitudinally spaced from said cylindrical lens.

As will be appreciated from the specific descriptions and explanations provided hereinafter, the present invention provides a new and useful laser chisel-like tool.

Described hereinafter is an exemplary embodiment of the invention which comprises an elongate pencil like tool having flexible fibre-optical connection at one end to the output of a laser emitting in the ultraviolet region of the spectrum. The fibre-optic transmission is expanded within the tool and converted to a collimated-light beam which is adapted to serve a plurality of different optical-projection configurations, each such configuration being unique to a different one of a plurality of projection fittings, detachably securable to the other end of the tool.

Further features of the invention are set forth with particularity in the appended claims and, together with the features described above, will become clear from consideration of the following description given in conjunction with the accompanying drawings, in which:

Fig. 1 is a simplified view in perspective, showing an exemplary surgical tool according to the invention, and further showing an associated laser device flexibly connected for supply of laser-output energy to the tool;

Fig. 2 is an enlarged longitudinal sectional view of the tool of Fig. 1; and

Fig. 3 illustrates an alternative projection fitting available for use with the tool of Fig. 2.

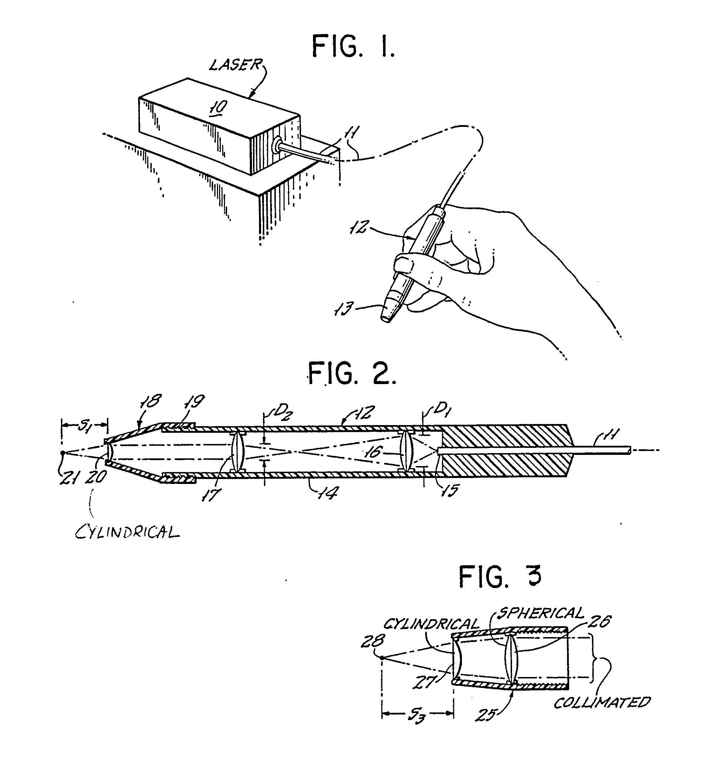

In Fig. 1, the invention is shown in conjunction with laser apparatus 10 producing an output beam via an optical-fiber cable 11 which provides flexible connection to the tail end of a hand-held manipulable tool 12. Tool 12 contains optical elements whereby the laser output delivered to tool 12 is converted at the forward or head end 13 of tool 12 for discharge as a ray bundle of the configuration desired for a particular surgical purpose.

More specifically, in Fig. 2 the tool 12 is seen to comprise an elongate generally tubular body 14 which is essentially closed at its tail end except for a reduced central bore via which the discharge end 15 of cable 11 is securely positioned. The radiation preferred in use of tool 12 is limited to the ultraviolet region of the spectrum, as for example the range wherein wavelength of the beam output of laser 10 is not more than substantially 400 nanometers (nm), illustratively in the range 150 nm to 400 nm. Such emissions for gas lasers are characteristically at 351 nm for xenon-fluoride lasers, 337 nm for nitrogen lasers, 308 nm for xenon-chloride lasers, 208 nm for krypton-fluoride lasers, 193 nm for argonfluoride lasers, and 157 nm for fluorine lasers; and within this range, frequency-doubling techniques applied to other lasers, including crystal lasers, provide further alternative sources.

One of the existing commercial excimer-laser products of Lambda Physik GmbH, Gottingen, Germany, for example their Model EMG 103 operating with argonfluoride, is satisfactory for use as laser 10. And in view of the short wavelength involved in the output of this laser, it is recommended that the optical fiber of cable 11 be of quartz, selected for optimum ultraviolet-transmission characteristics. This optical fiber will be understood to be suitably clad, and at the discharge end 15, the transversely truncated end of the optical fiber faces directly toward the head end of the tool and on the axis of the tubular cavity of body 14. Characteristically, the laser-emission discharge at 15 is divergent, and a first spherical lens 16 retained in body 14 receives the optical-fibre discharge (over a relatively large area, of diameter $D_1$) and converges the same to a smaller area (of diameter $D_2$) at a second spherical lens 17 which is also retained in body 14. The lens 17 is selected and positioned to establish projection of a collimated region of ultraviolet radiation on the tool axis and toward the head end.

For purposes of selective shaping of ultraviolet discharge from tool 12, provision is made for removable attachment of a final-lens mount or fitting 18 which is shown to be a generally frusto-conical tube having threaded engagement at 19 to the head end of body 14. A lens 20 retained at the reduced end of tube 17 is of a diameter to accept a full collimated bundle projected by lens 17; as shown, lens 20 is plano-cylindrical with its cylindrical axis projecting perpendicular to the plane of Fig. 2 and is of focal length selected to project a convergent ray bundle to a focal line at a desired projected longitudinal offset $S_1$ from lens 20. In view of the short wavelengths involved, it is preferred that all optical elements, such as lenses 16-17-20 be of quartz. In use, the surgeon's traversal of the focal line 21, along a body surface region of surgical operation, will be understood to achieve photodecomposition which results in a groove having sidewalls which diverge outwardly from a depth depending upon energy density and time of exposure.

It will be seen that, upon suitable choice of lens (17) focal length and positioning in body 14, the collimated-bundle diameter $D_2$ may be greater or less than implied by Fig. 2, so that the length of the line focus at 21 (and hence its energy density per unit length of the focal line) can be selected accordingly. The fitting 18 will be seen to provide the surgeon with an effective chisel which produces no offal or chips, enabling the surgeon to achieve a degree of smoothness in his manipulated sculpting of a given region of body tissue, such as the surface of a cornea.

The lens fitting 25 of Fig. 3 represents another alternative in place of the fitting 18 in Fig. 2. As indicated by legend, the optical contents of fitting 25 comprise a spherical lens 26 and a cylindrical lens 27, whereby the incoming collimated bundle projected by lens 17 is converted to focus at 28 and wherein the shape of the focal spot is elliptical rather than a line (Fig. 2).

It will be understood that in the embodiment of Fig. 3 the configuration of lenses within tool body 14 is such as to project a larger area bundle of collimated ultraviolet radiation to a short-focus cylindrical beam output lens. Due to the short focal length of the beam output lens, the angle of convergence of its projected ray bundle will be relatively large so that the energy density will be greatest at the focal region and relatively weak elsewhere. By appropriate adjustment of the laser beam energy, it can be arranged that the energy density at the focal region is sufficient to achieve photodecomposition of tissues such as the natural lens of and eye while in beam regions before the focal region it is insufficient to have any appreciable effect upon the corneal and/or retinal regions of the involved eye. Removal of a cataracted lens is thus possible by use of a laser tool according to the invention without surgical invasion of the eye.

It will be seen that the described tool provides the surgeon with an instrument which enables selective ablation of body tissue without charring any body cells, and the ablation may be performed either upon a region of the outer surface of the body or beneath the outer surface as in the case of removal of a cataracted natural lens.

## Claims

1. A tool for use in laser surgery, said tool (12) being adapted to be connected to *laser means (10) having a radiation-beam output by means of an elongate flexible optical-fibre cable (11) coupled at one end to said output and at the other end to said tool, said tool (12) having a body (14) with a cavity bore to one end of which the said other end of said cable (11) is affixed in use for discharge of the laser output into the cavity bore of the tool, optical element means (16, 17) mounted in said bore for establishing a collimated beam of laser radiation directed towards the other end of the bore, and a lens mount (18; 25) separate from but attachable to the body (14) at the other end of the bore and incorporating lens means (20; 27) for focussing said beam of laser radiation, characterized in that said lens means (20; 27) comprises a cylindrical lens mounted with its axis transverse to the axis of said bore for focussing the beam of laser radiation into an elongate region transverse to said axis and longitudinally spaced from said cylindrical lens.

2. A tool as claimed in claim 1, in which the cylindrical lens (20; 27) is arranged to focus said collimated beam to a line transverse to said axis and longitudinally spaced from the cylindrical lens.

3. A tool as claimed in claim 1 in which a spherical lens (26) is positioned in axial advance of the cylindrical lens (27) in said lens mount (25) for generating a convergent beam which impinges upon said cylindrical lens (27) and is focussed thereby into an elongate region of elliptical area.

4. A tool as claimed in any preceding claim wherein said cylindrical lens comprises a plano-cylindrical lens.

5. A tool as claimed in any of the preceding claims wherein said optical elements means (16, 17) mounted in said bore of the tool body is adapted to establish a collimated beam of laser radiation having a transverse dimension which is expanded as compared to the discharge face dimension of the optical fibre cable.

6. Apparatus for laser surgery comprising, in combination, laser means (10) having a radiation-beam output, an elongate flexible optical-fibre cable (11) coupled at one end to said output, and a tool (12) as claimed in any of the preceding claims coupled to the other end of said cable (11).

7. An apparatus as claimed in claim 6, in which said laser means (10) is an ultraviolet laser adapted to produce a radiation beam output having a wavelength of not more than about 400 nm.

8. An apparatus as claimed in claim 6 or 7, in which the coupling of said cable (11) to said laser output includes a beam splitter, and a source of visible light is also coupled to said cable (11) via said beam splitter.

9. An apparatus as claimed in claim 6 or 7 or 8, in which the optical fibre of said cable is of quartz.

## Patentansprüche

1. Instrument zur Anwendung in der Laser-Chirurgie, wobei das Instrument (12) mit einer Laser-Einrichtung (10), die einen Ausgang für einen Strahlungsstrahl hat, mittels eines langgestreckten, flexiblen optischen Faserkabels (11), das am einen Ende an den Ausgang und am anderen Ende an das Instrument angeschlossen ist, zu verbinden ist, das Instrument (12) einen Körper (14) mit einer Hohlraumbohrung, an deren einem Ende das andere Ende des Kabels (11) im Gebrauch für ein Einführen des Laserausgangs in die Hohlraumbohrung des Instruments befestigt ist, aufweist, optische Elemente (16, 17) in diese Bohrung eingebaut sind, um einen kollimierten, zum anderen Ende der Bohrung hin gerichteten Strahl einer Laserstrahlung zu bilden, und einer vom Körper (14) getrennten, jedoch am anderen Ende der Bohrung anbaubaren Linsenfassung (18; 25) Linseneinrichtungen (20; 27) zur Fokussierung des Strahls der Laserstrahlung eingegliedert sind, dadurch gekennzeichnet, daß die Linseneinrichtungen (20; 27) eine Zylinderlinse umfassen, die mit ihrer Achse quer zur Achse der Bohrung eingebaut ist, um den Strahl der Laserstrahlung in einen verlängerten, zur genannten Achse transversalen und in Längsrichtung von der Zylinderlinse beabstandeten Bereich zu fokussieren.

2. Instrument nach Anspruch 1; wobei die Zylinderlinse (20; 27) zur Fokussierung des kollimierten Strahls aufeine.

3. Instrument nach Anspruch 1, wobei eine sphärische Linse (26) in axialer Richtung vor der Zylinderlinse (27) in der Linsenfassung (25) angeordnet ist, um einen konvergenten Strahl zu erzeugen, der auf die Zylinderlinse (27) trifft und dadurch in einen verlängerten Bereich mit elliptischer Fläche fokussiert wird.

4. Instrument nach einem der vorhergehenden Ansprüche, wobei die Zylinderlinse eine Planzylinderlinse ist.

5. Instrument nach einem der vorhergehenden Ansprüche, wobei die in die Bohrung des Instrumentkörpers eingebauten Linseneinrichtungen (16, 17) imstande sind, einen kollimierten Strahl einer Laserstrahlung, der eine transversale, im Vergleich zur Abmessung der Austrittsfläche des optischen Faserkabels erweiterte Abmessung hat, zu bilden.

6. Vorrichtung zur Laser-Chirurgie, die in Kombination umfaßt eine Lasereinrichtung (10) mit einem Strahlungsstrahlausgang, ein langgestrecktes, flexibles optisches Faserkabel (11), das am einen Ende an den genannten Ausgang angeschlossen ist, und ein Instrument (12), das in einem der vorhergehenden Ansprüche beansprucht sowie an das andere Ende dieses Kabels (11) angeschlossen ist.

7. Vorrichtung nach Anspruch 6, wobei die Lasereinrichtung (10) ein Ultraviolettlaser ist, der imstande ist, einen Strahlungsstrahl mit einer Wellenlänge von nicht mehr als etwa 400 nm zu erzeugen.

8. Vorrichtung nach Anspruch 6 oder 7, wobei der Anschluß des Kabels (11) an den Laserausgang einen Strahlenteiler enthält und eine Quelle von sichtbarem Licht ebenfalls über diesen Strahlenteiler an das genannte Kabel (11) angeschlossen ist.

9. Vorrichtung nach Anspruch 6 oder 7 oder 8, wobei die optische Faser des Kabels aus Quarz besteht.

## Revendications

1. Outil utilisable en chirurgie au laser, ledit outil (12) étant adapté pour être relié à un moyen laser (10) possédant une sortie pour un faisceau de rayonnement, au moyen d'un câble à fibre optique flexible allongé (11) couplé, par une extrémité, à ladite sortie et, par la seconde extrémité, audit outil, ledit outil (12) possédant un corps (14) avec un alésage en forme de cavité à une extrémité duquel est fixée, en service, ladite seconde extrémité dudit câble (11) pour décharger la sortie du laser dans l'alésage en forme de cavité de l'outil, des moyens formant éléments optiques (16, 17) montés dans ledit alésage pour établir un faisceau collimaté de rayonnement laser dirigé vers la seconde extrémité de l'alésage, et une monture de lentilles (18, 25) séparée du corps (14), mais susceptible d'y être attaché à la seconde extrémité de l'alésage, et renfermant des moyens formant lentilles (20; 27) pour focaliser ledit faisceau de rayonnement laser, caractérisé en ce que lesdits moyens formant lentilles (20; 27) comprennent une lentille cylindrique montée avec son axe transversal à l'axe dudit alésage pour focaliser le faisceau de rayonnement laser dans une région allongée transversale audit axe et distante de ladite lentille cylindrique dans la direction longitudinale.

2. Outil tel que défini dans la revendication 1, dans lequel la lentille cylindrique (20; 27) est disposée pour focaliser ledit faisceau collimaté sur une ligne transversale audit axe et distante de la lentille cylindrique dans la direction longitudinale.

3. Outil tel que défini dans la revendication 1, dans lequel une lentille sphérique (26) est positionnée axialement en avant de la lentille cylindrique (27) dans ladite monture de lentille (25) pour engendrer un faisceau convergent qui vient frapper ladite lentille cylindrique (27) et est focalisé par celle-ci dans une région allongée de surface elliptique.

4. Outil tel que défini dans l'une quelconque des revendications précédentes, dans lequel ladite lentille cylindrique comprend une lentille plan-cylindrique.

5. Outil el que défini dans l'une quelconque des revendications précédentes, dans lequel lesdits moyens (16, 17) formant éléments optiques montés dans ledit alésage du corps de l'outil sont adaptés pour établir un faisceau collimaté de rayonnement laser possédant une dimension transversale qui est dilatée par rapport à la dimension de la face de décharge du câble à fibre optique.

6. Appareil destiné à la chirurgie au laser comprenant, en combinaison, un moyen laser (10) possédant une sortie Pour un faisceau de rayonnement, un câble (11) à fibre optique flexible allongé couplé, par une extrémité, à ladite sortie, et un outil (12) selon l'une quelconque des revendications précédentes couplé à l'autre extrémité dudit câble (11).

7. Appareil tel que défini dans la revendication 6, dans lequel ledit moyen laser (10) est un laser ultra-violet adapté pour produire un faisceau de rayonnement de sortie possédant une longueur d'onde non supérieure à environ 400 nm.

8. Appareil tel que défini dans la revendication 6 ou 7, dans lequel le couplage dudit câble (11) à ladite sortie du laser comprend un séparateur de faisceau, et une source de lumière visible est également couplée audit câble (11) par l'intermédiaire dudit séparateur de faisceau.

9. Appareil tel que défini dans la revendication 6 ou 7 ou 8, dans lequel la fibre optique dudit câble est en quartz.

# FIG. I.

LASER

*10*

*11*

*12*

*13*

# FIG. 2.

*18*  *19*  $D_2$  *12*  $D_1$  *11*

*21 20*  *17*  *14*  *16*  *15*

$S_1$

CYLINDRICAL

# FIG. 3

CYLINDRICAL  SPHERICAL  *26*

*28*

*27*  *25*  COLLIMATED

$S_3$